(19) Europäisches Patentamt
European Patent Office
Office européen des brevets

(11) Veröffentlichungsnummer: **0 176 846 B1**

# (12) EUROPÄISCHE PATENTSCHRIFT

(45) Veröffentlichungstag der Patentschrift: **16.08.89**

(51) Int. Cl.⁴: **C 07 C 101/34**, C 07 C 103/30, C 07 C 121/78, C 07 D 295/14

(21) Anmeldenummer: **85111670.7**

(22) Anmeldetag: **16.09.85**

## (54) 3-Amino-2-benzoyl-acrylsäurederivate und ein Verfahren zu ihrer Herstellung.

(30) Priorität: **29.09.84 DE 3435930**
**30.01.85 DE 3502935**

(43) Veröffentlichungstag der Anmeldung:
**09.04.86 Patentblatt 86/15**

(45) Bekanntmachung des Hinweises auf die Patenterteilung:
**16.08.89 Patentblatt 89/33**

(84) Benannte Vertragsstaaten:
**AT BE CH DE FR GB IT LI NL SE**

(56) Entgegenhaltungen:
**EP-A- 0 016 277**
**EP-A- 0 078 362**
**EP-A- 0 129 846**
**DE-A- 2 330 913**
**US-A- 4 243 406**

**Die Akte enthält technische Angaben, die nach dem Eingang der Anmeldung eingereicht wurden und die nicht in dieser Patentschrift enthalten sind.**

(73) Patentinhaber: **BAYER AG,**
**D-5090 Leverkusen 1 Bayerwerk (DE)**

(72) Erfinder: **Grohe, Klaus, Dr., Am Wasserturm 10,**
**D-5068 Odenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die vorliegende Erfindung betrifft 3-Amino-2-benzoyl-acrylsäurederivate, die wertvolle Zwischenprodukte für die Synthese von hochwirksamen antibakteriellen Arzneimitteln aus der Gruppe der Chinoloncarbonsäuren sind, und ein Verfahren zu ihrer Herstellung.

Aus der US-PS 4 243 406 ist Ethyl-2-(2′,4′-dichlorobenzoyl)-3-dimethylaminopropenoat als Zwischenprodukt für herbizide Verbindungen bekannt.

Es wurde gefunden, dass man 3-Amino-2-benzoyl-acrylsäurederivate der Formel I

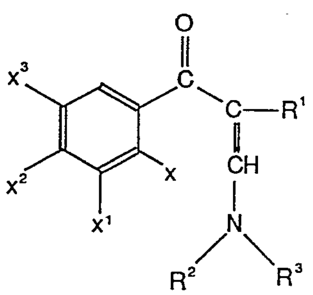

I

in welcher

$R^1$ für die Nitrilgruppe, eine Estergruppe $-COOR^4$ oder Carbonamidgruppe

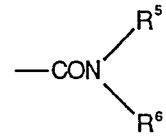

steht, wobei

$R^4$ $C_1$-$C_6$-Alkyl bedeuten und

$R^5$ und $R^6$ für Wasserstoff, $C_1$-$C_3$-Alkyl oder Phenyl stehen,

$R^2$ und $R^3$ gleich oder verschieden sein können und für einen $C_1$-$C_6$-Alkylrest stehen und weiterhin gemeinsam mit dem Stickstoffatom, an das sie gebunden sind, einen 5- oder 6-gliedrigen heterocyclischen Ring bilden können, der als Ringglied zusätzlich die Atome oder Gruppen $-O-$, $-S-$, $-SO-$ oder $-SO_2-$ enthalten kann,

X für Halogen, bevorzugt Chlor oder Fluor, steht,

$X^1$ Wasserstoff, Methyl, Nitro oder Halogen, bevorzugt Fluor, bedeutet,

$X^2$ Halogen, bevorzugte Chlor oder Fluor, oder Methyl sein kann und

$X^3$ Wasserstoff oder Halogen, bevorzugt Fluor, darstellt,

ausgenommen Ethyl-2-(2′,4′-dichlorobenzoyl)-3-dimethylaminopropenoat erhält, wenn man Benzoylhalogenide der Formel II, in welcher Hal bevorzugt Chlor oder Brom bedeutet und X, $X^1$, $X^2$ sowie $X^3$ die oben angegebene Bedeutung haben, mit 3-Amino-acrylsäurederivaten der Formel III, in welcher $R^1$, $R^2$ und $R^3$ die oben angegebene Bedeutung haben, gegebenenfalls in Gegenwart einer Base, umsetzt:

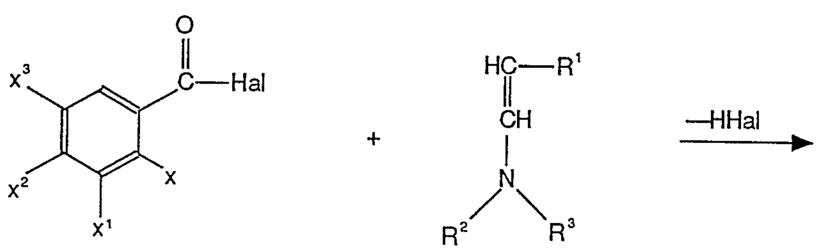


I

II III

Die erfindungsgemässen 3-Amino-2-benzoyl-acrylsäurederivate 1, die, ausgenommen Ethyl-2-(2′-,4′-dichlorobenzoyl)-3-dimethyl-aminopropenoat ebenfalls Gegenstand dieser Erfindung sind, können mit Cyclopropylamin IV in die teilweise bekannten, bereits auf anderem Wege synthetisierten 3-Cyclopropyl-amino-2-benzoyl-acrylsäurederivate V überführt werden:

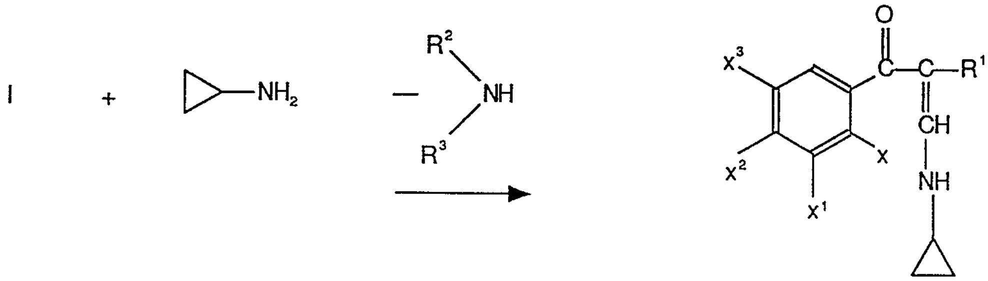

IV V

Es ist bereits bekannt geworden, dass man Monoalkyl-amino-acrylsäurederivate III ($R^1$ = $COOR^4$, $R^2$ = H) mit Benzoylhalogeniden II acyliert (europäisches Patent 0 004 279), wobei teilweise N-Acylierung eintritt. Das erfindungsgemässe Verfahren hat dagegen den Vorteil, dass nur die C-Acylierung des Enamin-Systems erfolgt, was zu einer beträchtlichen Ausbeuteerhöhung führt.

Verwendet man 2,4-Dichlor-5-fluor-benzoylchlorid (1) und 3-Dimethylamino-acrylsäuremethylester (2) als Ausgangsstoffe, so kann der erfindungsgemässe Reaktionsablauf durch das folgende Formelschema wiedergegeben werden:

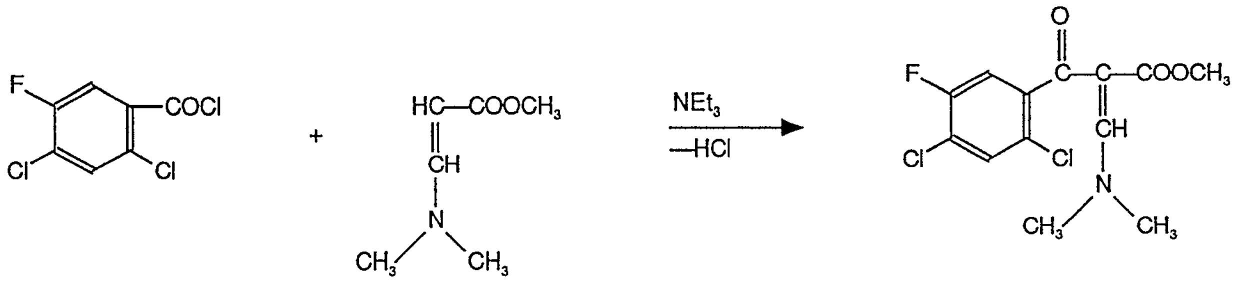


1 2 3

Bei der weiteren Umsetzung von (3) mit Cyclopropylamin entsteht der 3-Cyclopropylaminoacrylsäureester (4), der dann in das antibakterielle Breitbandchemotherapeutikum Ciprofloxacin (vgl. DE-OS 3 142 854) überführt werden kann:

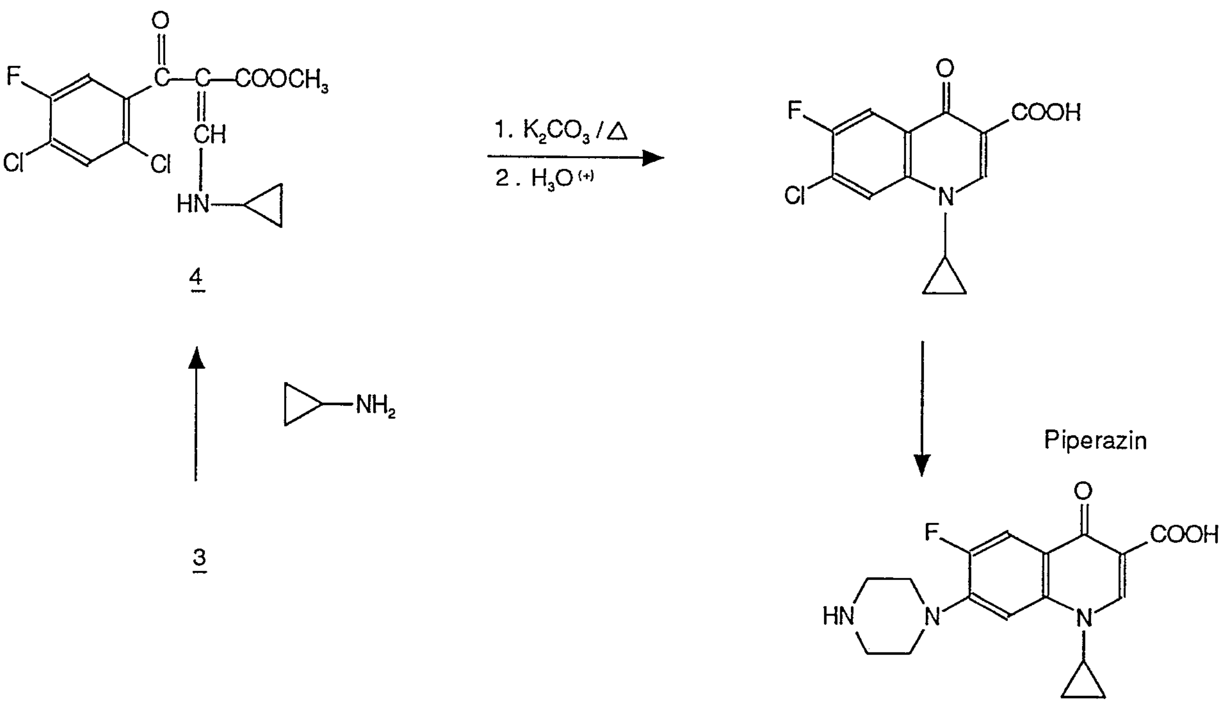


4

3

Ciprofloxacin

Die erfindungsgemäss verwendbaren fluor- und chlorhaltigen Benzoylhalogenide II oder die entsprechenden Carbonsäuren sind bekannt, z.B. aus DE-OS 3 142 854.

Als Beispiele seien genannt:
2,4-Dichlor-5-fluor-benzoylchlorid, 2,4,5-Trifluor-benzoylchlorid, 2,3,4,5-Tetrafluor-benzoylchlorid, 2,4,5-Trichlorbenzoylchlorid, 2,3,4,5-Tetrachlorbenzoylchlorid, 2,4,5-Trifluor-3-chlorbenzoylchlorid. Gleichfalls bekannt sind die 3-Amino-acrylsäurederivate III.

Als Beispiele seien genannt:
3-Dimethylaminoacrylsäuremethylester, 3-Dimethylamino-acrylsäureethylester, 3-Dimethylamino-acrylsäurenitril, 3-Dimethylamino-acrylsäureamid, 3-Dimethylamino-acrylsäure-methylamid, 3-Dimethylamino-acrylsäure-dimethylamid, 3-Dimethylamino-acrylsäureanilid.

Die Umsetzung der halogenierten Benzoylhalogenide II mit den 3-Aminoacrylsäurederivaten III wird vorzugsweise in einem inerten Verdünnungsmittel vorgenommen. In Frage kommen Methylenchlorid, Chloroform, Toluol, Tetrahydrofuran und Dioxan.

Die Umsetzung wird bei Temperaturen zwischen 10 und 200°C vorzugsweise zwischen 20 und 110°C, durchgeführt. Es wird bevorzugt bei Normaldruck gearbeitet.

Die Reaktionspartner II und III werden bevorzugt im stöchiometrischen Verhältnis 1:1 eingesetzt.

Als Säureakzeptor werden bevorzugt Pyridin, Triethylamin, N-Methylpiperidin sowie Natriumhydrid verwendet.

Die Umsetzung der 3-Amino-benzoyl-acrylsäurederivate I mit Cyclopropylamin IV zu V wird bevorzugt in einem Verdünnungsmittel wie z. B. Cyclohexan, Toluol, Dioxan, Tetrachlorkohlenstoff oder Chlorbenzol bei Temperaturen von 10 bis 200°C, bevorzugt 20 bis 120°C vorgenommen.

Die erfindungsgemässen Verbindungen sind wertvolle Zwischenprodukte für die Herstellung von hochwirksamen antibakteriellen Arzneimitteln, die beispielsweise in den DE-OSen 3 033 157 und 3 142 854 beschrieben werden.

Beispiel 1

3-Dimethylamino-2-(2,4-dichlor-5-fluor-benzoyl)-acryl-säuremethylester

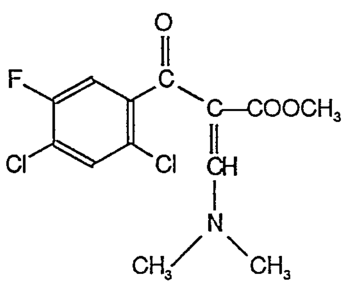

Eine Lösung von 22,75 g 2,4-Dichlor-5-fluor-benzoyl-chlorid in 80 ml wasserfreiem Dioxan wird unter Eiskühlung und Rühren zunächst tropfenweise mit einer Lösung von 12,9 g 3-Dimethylamino-acrylsäuremethylester in 25 ml Dioxan und anschliessend 10,5 g Triethylamin versetzt. Man rührt 3 Stunden bei Raumtemperatur, erhitzt 1 Stunde auf 50–60°C, destilliert das Lösungsmittel im Vakuum ab und nimmt den Rückstand in Methylenchlorid/$H_2O$ auf. Die Phasen werden getrennt und die wässrige Lösung mit Methylenchlorid nachextrahiert. Die vereinigten organischen Phasen werden mit Wasser gewaschen, mit Natriumsulfat getrocknet und das Methylenchlorid im Vakuum abgezogen. Der kristalline Rückstand wird aus Methanol/Wasser umkristallisiert. Es werden 28,5 g 3-Dimethylamino-2-(2,4-dichlor-5-fluor-benzoyl)-acrylsäuremethylester vom Schmelzpunkt 107–109°C erhalten.

Beispiel 2

3-Dimethylamino-2-(2,4-dichlor-5-fluor-benzoyl)-acryl-säureethylester

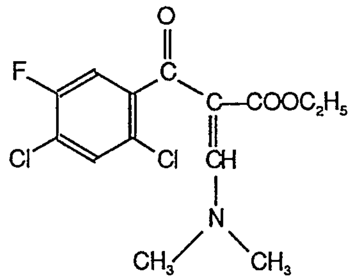

Eine Lösung von 22,75 g 2,4-Dichlor-5-fluor-benzoyl-chlorid in 100 ml wasserfreiem Dioxan wird bei 10 bis 20°C unter Rühren tropfenweise mit 14,3 g 3-Dimethyl-amino-acrylsäureethylester und 10,5 g Triethylamin versetzt. Man rührt 3 Stunden bei Raumtemperatur, erhitzt 1 Stunde auf 40–50°C, destilliert das Lösungsmittel im Vakuum ab und nimmt den Rückstand in Methylenchlorid/$H_2O$ auf. Die Phasen werden getrennt und die wässrige Lösung mit Methylenchlorid nachextrahiert. Die $CH_2Cl_2$-Lösung wird mit Wasser gewaschen, mit Natriumsulfat getrocknet und das Lösungsmittel im Vakuum abgezogen. Der kristalline Rückstand wird aus Cyclohexan/Leichtbenzin umkristallisiert. Es werden 27,8 g 3-Dimethylamino-2-(2,4-dichlor-5-fluor-benzoyl)-acrylsäureethylester vom Schmelzpunkt 94–95°C erhalten.

Die nachstehend beschriebene Umsetzung dieser Verbindung mit Cyclopropylamin führt zum 3-Cyclopropylamino-2-(2,4-dichlor-5-fluorbenzoyl)-acrylsäureethylester:

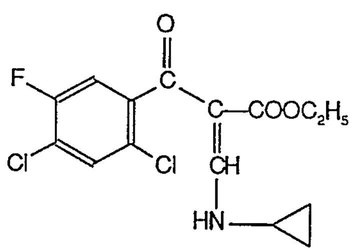

33,4 g 3-Dimethylamino-2-(2,4-dichlor-5-fluorbenzoyl)-acrylsäureethylester werden mit 7 g Cyclopropylamin und 120 ml Toluol eine Stunde unter Rückfluss zum Sieden erhitzt. Die zunächst stürmisch einsetzende Gasentwicklung ist dann beendet. Man destilliert das Toluol im Vakuum ab und kristallisiert den festen Rückstand aus Leichtbenzin um. Es werden 32,5 g 3-Cyclopropylamino-2-(2,4-dichlor-5-fluor-benzoyl)-acrylsäureethylester vom Schmelzpunkt 89–91°C erhalten.

Beispiel 3

3-Dimethylamino-2-(2,4-dichlor-5-fluor-benzoyl)-acrylsäurenitril

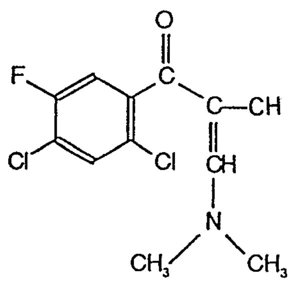

Eine Lösung von 22,75 g 2,4-Dichlor-5-fluor-benzoyl-chlorid in 100 ml wasserfreiem Dioxan wird unter Eiskühlung und Rühren tropfenweise mit 9,6 g 3-Dimethyl-amino-acrylnitril und 10,5 g Triethylamin versetzt. Man rührt eine Stunde bei Raumtemperatur und erhitzt anschliessend 4 Stunden unter Rückfluss zum Sieden. Das Lösungsmittel wird im Vakuum abdestilliert, der Rückstand in Methylenchlorid/Wasser aufgenommen. Die Methylenchlorid-Phase wird mit Wasser gewaschen mit Natriumsulfat getrocknet und im Vakuum eingeengt. Der kristalline Rückstand wird aus Ethanol umkristallisiert. Es werden 22,2 g 3-Dimethylamino-2-(2,4-dichlor-5-fluor-benzoyl)-acrylsäurenitril vom Schmelzpunkt 138–139 °C erhalten.

Die nachstehend beschriebene Umsetzung dieser Verbindung mit Cyclopropylamin führt zum 3-Cyclopropylamino-2-(2,4-dichlor-5-fluorbenzoyl)-acrylsäurenitril:

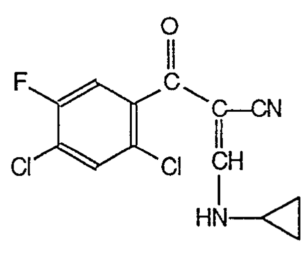

14,35 g 3-Dimethylamino-2-(2,4-dichlor-5-fluorbenzoyl)-acrylsäurenitril werden mit 3,2 g Cyclopropylamin und 60 ml Toluol refluxiert. Wenn die Gasentwicklung nach ca. 30 Minuten beendet ist wird das Toluol im Vakuum abdestilliert und der Rückstand aus Ethanol/Leichtbenzin umkristallisiert. Es werden 13,8 g 3-Cyclopropylamino-2-(2,4-dichlor-5-fluorbenzoyl)-acrylsäurenitril vom Schmelzpunkt 94–95 °C erhalten.

Beispiel 4

3-Dimethylamino-2-(2,4-dichlor-5-fluor-benzoyl)-acrylsäure-dimethylamid

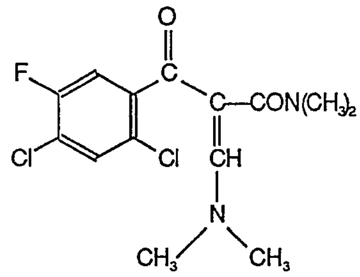

Eine Lösung von 22,75 g 2,4-Dichlor-5-fluor-benzoylchlorid in 100 ml wasserfreiem Dioxan wird unter Eiskühlung und Rühren zunächst portionsweise mit 14,3 g 3-Dimethylaminoacrylsäuredimethylamid und dann tropfenweise mit 10,5 g Triethylamin versetzt. Man rührt 3 Stunden bei Raumtemperatur und erhitzt dann weitere 3 Stunden auf 50–60 °C. Das Lösungsmittel wird im Vakuum abgezogen und der Rückstand zwischen Methylenchlorid und Wasser verteilt. Die Methylenchlorid-Lösung wird mit Wasser gewaschen, mit Natriumsulfat getrocknet und das Lösungsmittel im Vakuum abgezogen. Der kristalline Rückstand wird aus Ethanol/Wasser umkristallisiert. Es werden 21,5 g 3-Dimethylamino-2-(2,4-dichlor-5-fluor-benzoyl)-acrylsäuredimethylamid vom Schmelzpunkt 124–126 °C erhalten.

Beispiel 5

3-(1-Pyrrolidinyl)-2-(2,4-dichlor-5-fluor-benzoyl)-acrylsäuremethylester

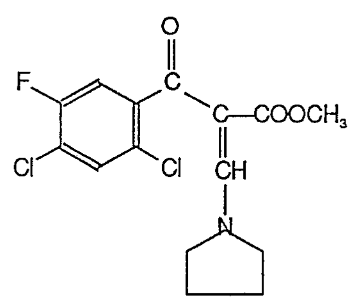

Eine Lösung von 22,75 g 2,4-Dichlor-5-fluor-benzoylchlorid in 80 ml wasserfreiem Dioxan wird unter Eiskühlung und Rühren zunächst tropfenweise mit einer Lösung von 15,5 g 3-(1-Pyrrolidinyl)-acrylsäuremethylester in 25 ml Dioxan und anschliessend mit 10,5 g Triethylamin versetzt. Man rührt 1 Stunde bei Raumtemperatur, erhitzt 30 Minuten unter Rückfluss zum Sieden, destilliert das Lösungsmittel im Vakuum ab und nimmt den Rückstand in Methylenchlorid/$H_2O$ auf. Die Phasen werden getrennt und die wässrige Lösung mit Methylenchlorid nachextrahiert. Die vereinigten organischen Phasen werden mit Wasser gewaschen, mit Natriumsulfat getrocknet und das Methylenchlorid im Vakuum abgezogen. Der kristalline Rückstand wird aus Cyclohexan umkristallisiert. Es werden 19,6 g 3-(1-Pyrroli-

dinyl)-2-(2,4-dichlor-5-fluorbenzoyl)-acrylsäuremethylenester vom Schmelzpunkt 74–76 °C erhalten.

Die nachstehend beschriebene Umsetzung dieser Verbindung mit Cyclopropylamin führt zum 3-Cyclopropylamino-2-(2,4-dichlor-5-fluorbenzoyl)-acrylsäuremethylester

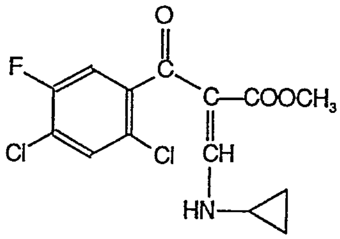

3,5 g 3-(1-Pyrrolidinyl)-2-(2,4-dichlor-5-fluorbenzoyl)-acrylsäuremethylester werden mit 0,8 g Cyclopropylamin und 50 ml Toluol eine Stunde unter Rückfluss zum Sieden erhitzt. Man destilliert das Toluol im Vakuum ab und kristallisiert den Rückstand aus Acetonitril um. Es werden 2,5 g 3-Cyclopropylamino-2-(2,4-dichlor-5-fluor-benzoyl)-acrylsäuremethylester vom Schmelzpunkt 150–151 °C erhalten.

Beispiel 6

3-Diethylamino-2-(2,4-dichlor-5-fluor-benzoyl)-acrylsäureethylester

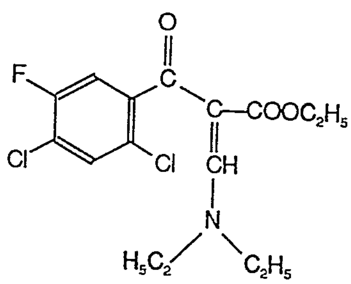

Eine Lösung von 22,75 g 2,4-Dichlor-5-fluor-benzoylchlorid in 80 ml wasserfreiem Dioxan wird unter Eiskühlung und Rühren zunächst tropfenweise mit 17,1 g 3-Diethylamino-acrylsäureethylester und anschliessend mit 10,5 g Triethylamin versetzt. Man rührt eine Stunde bei Raumtemperatur, erhitzt 45 Minuten unter Rückfluss zum Sieden, destilliert das Lösungsmittel im Vakuum ab und nimmt den öligen Rückstand in Methylenchlorid/Wasser auf. Die Phasen werden getrennt und die wässrige Lösung mit Methylenchlorid nachextrahiert. Die vereinigten organischen Phasen werden mit Wasser gewaschen, mit Natriumsulfat getrocknet und das Methylenchlorid im Vakuum abgezogen. Es werden 29 g 3-Diethylamino-2-(2,4-dichlor-5-fluor-benzoyl)-acrylsäureethylester als braunes Öl erhalten.

Die nachstehend beschriebene Umsetzung dieser Verbindung mit Cyclopropylamin führt zum 3-Cyclopropylamino-2-(2,4-dichlor-5-fluorbenzoyl)-acrylsäureethylester:

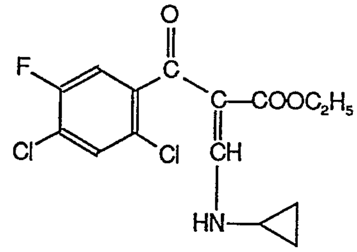

17,5 g 3-Diethylamino-2-(2,4-dichlor-5-fluor-benzoyl)-acrylsäureethylester werden mit 2,9 g Cyclopropylamin und 50 ml Toluol 30 Minuten unter Rückfluss zum Sieden erhitzt. Das Toluol wird im Vakuum abdestilliert und der kristallin erstarrende Rückstand aus Cyclohexan/Leichtbenzin umkristallisiert. Es werden 12,9 g 3-Cyclopropyl-amino-2-(2,4-dichlor-5-fluor-benzoyl)-acrylsäureethylester vom Schmelzpunkt 89–90 °C erhalten.

Beispiel 7

3-Dimethylamino-2-(2,3,4,5-tetrafluor-benzoyl)-acrylsäurenitril

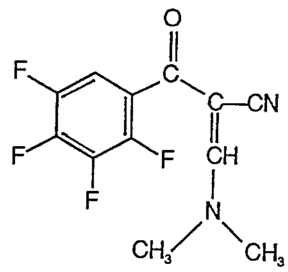

Eine Lösung von 21,25 g 2,3,4,5-Tetrafluorbenzoylchlorid in 75 ml wasserfreiem Dioxan wird unter Eiskühlung nach Rühren bei etwa 10–15 °C zunächst tropfenweise mit 9,7 g 3-Dimethylamino-acrylnitril und anschliessend mit 10,5 g Triethylamin versetzt. Man erhitzt 4 Stunden unter Rückfluss zum Sieden, destilliert das Lösungsmittel im Vakuum ab und nimmt den Rückstand in Methylenchlorid/Wasser auf. Die Phasen werden getrennt und die wässrige Lösung mit Methylenchlorid nachextrahiert. Die vereinigten organischen Phasen werden mit Wasser gewaschen, mit Natriumsulfat getrocknet und das Methylenchlorid im Vakuum abgezogen. Nach Umkristallisation des kristallinen Rückstands aus Ethanol werden 23,5 g 3-Dimethylamino-2-(2, 3, 4, 5-tetrafluor-benzoyl)-acrylsäurenitril vom Schmelzpunkt 149–151 °C erhalten.

**Patentansprüche**

1. 3-Amino-2-benzoyl-acrylsäurederivate der Formel I

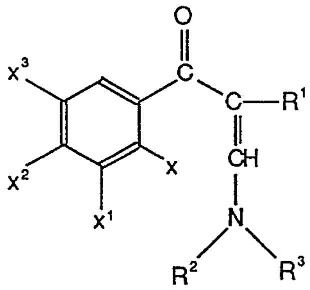

I

in welcher
$R^1$ für die Nitrilgruppe, eine Estergruppe $-COOR^4$ oder Carbonamidgruppe

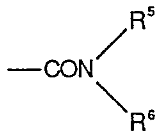

steht, wobei
$R^4$ $C_1$-$C_6$-Alkyl bedeutet und
$R^5$ und $R^6$ für Wasserstoff, $C_1$–$C_3$-Alkyl oder Phenyl stehen,
$R^2$ und $R^3$ gleich oder verschieden sein können und für einen $C_1$-$C_6$-Alkylrest stehen und weiterhin gemeinsam mit dem Stickstoffatom, an das sie gebunden sind, einen 5- oder 6-gliedrigen heterocyclischen Ring bilden können, der als Ringglied zusätzlich die Atome oder Gruppen -O-, -S-, -SO- oder $-SO_2-$ enthalten kann,

X für Halogen steht,
$X^1$ Wasserstoff, Methyl, Nitro oder Halogen bedeutet,
$X^2$ Halogen, Methyl und
$X^3$ Wasserstoff oder Halogen bedeutet,

ausgenommen Ethyl-2-(2',4'-dichlorobenzoyl)-3-dimethylaminopropenoat.

2. 3-Dimethylamino-2-(2,4-dichlor-5-fluorbenzoyl)-acrylsäure-methylester.

3. 3-Dimethylamino-2-(2,4-dichlor-5-fluorbenzoyl)-acrylsäure-ethylester.

4. 3-Dimethylamino-2-(2,3,4,5-tetrafluor-benzoyl)-acrylsäurenitril.

5. 3-Dimethylamino-2-(2,4-dichlor-5-fluorbenzoyl)-acrylsäurenitril.

6. Verfahren zur Herstellung von 3-Amino-2-benzoyl-acrylsäurederivaten der Formel I

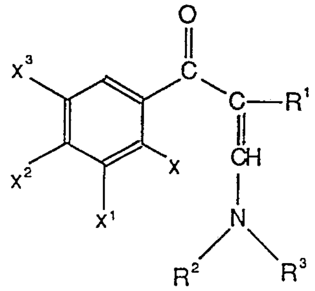

I

in welcher
$R^1$ für die Nitrilgruppe, eine Estergruppe $-COOR^4$ oder Carbonamidgruppe

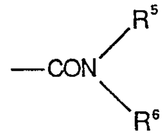

steht, wobei

$R^4$ $C_1$-$C_6$-Alkyl bedeutet und
$R^5$ und $R^6$ für Wasserstoff, $C_1$-$C_3$-Alkyl oder Phenyl stehen,
$R^2$ und $R^3$ gleich oder verschieden sein können und für einen $C_1$-$C_6$-Alkylrest stehen und weiterhin gemeinsam mit dem Stickstoffatom, an das sie gebunden sind, einen 5- oder 6-gliedrigen heterocyclischen Ring bilden können, der als Ringglied zusätzlich die Atome oder Gruppen -O-, -S-, -SO- oder $-SO_2-$ enthalten kann,
X für Halogen steht,
$X^1$ Wasserstoff, Methyl, Nitro oder Halogen bedeutet,
$X^2$ Halogen, Methyl und
$X^3$ Wasserstoff oder Halogen bedeutet, ausgenommen Ethyl-2-(2',4'-dichlorobenzoyl)-3-dimethyl-aminopropenoat,
dadurch gekennzeichnet, dass man Benzoylhalogenide der Formel II

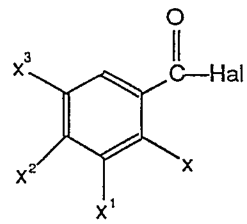

II

in welcher Hal Halogen bedeutet und X, $X^1$, $X^2$ und $X^3$ die oben angegebene Bedeutung haben, mit 3-Aminoacrylsäurederivaten der Formel III

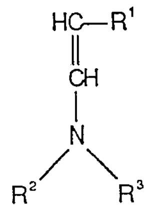

III

in welcher $R^1$, $R^2$ und $R^3$ die oben angegebene Bedeutung haben, gegebenenfalls in Gegenwart eines Säureakzeptors umsetzt.

7. Verfahren nach Anspruch 6, dadurch gekennzeichnet, dass man 2,4-Dichlor-5-fluor-benzoylchlorid mit 3-Dimethylamino-acrylsäuremethylester umsetzt.

8. Verfahren nach Anspruch 6, dadurch gekennzeichnet, dass man 2,4-Dichlor-5-fluor-benzoylchlorid mit 3-Dimethylamino-acrylsäureethylester umsetzt.

9. Verfahren nach Anspruch 6, dadurch gekennzeichnet, dass man 2,4-Dichlor-5-fluor-benzoylchlorid mit 3-Dimethylamino-acrylnitril umsetzt.

10. Verfahren nach Anspruch 6, dadurch gekennzeichnet, dass man als Säureakzeptor eine organische Base oder Natriumhydrid verwendet.

11. Verwendung der 3-Amino-2-benzoyl-acrylsäurederivate der Formel I in Anspruch 1 einschliesslich der in Anspruch 1 ausgenommenen Verbindung als Zwischenprodukte für die Synthese von antibakteriellen Arzneimitteln aus der Gruppe der Chinoloncarbonsäuren.

**Claims**

1. 3-Amino-2-benzoylacrylic acid derivatives of the formula I

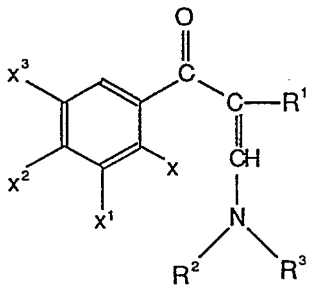

I

in which

$R^1$ represents the nitrile group, an ester group $-COOR^4$ or carboxamide group

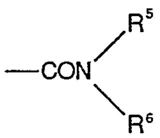

$R^4$ denoting $C_1$–$C_6$-alkyl, and

$R^5$ and $R^6$ representing hydrogen or $C_1$–$C_3$-alkyl or phenyl,

$R^2$ and $R^3$, which can be identical or different, represent a $C_1$–$C_6$-alkyl radical and, furthermore, can form, together with the nitrogen atom to which they are bonded, a 5- or 6-membered heterocyclic ring which can additionally contain as ring member the atoms or groups $-O-$, $-S-$, $-SO-$ or $-SO_2-$,

X represents halogen,

$X^1$ denotes hydrogen, methyl, nitro or halogen,

$X^2$ denotes halogen or methyl, and

$X^3$ denotes hydrogen or halogen,

excluding ethyl 2-(2′,4′-dichlorobenzoyl)-3-dimethylaminopropenoate.

2. Methyl 3-dimethylamino-2-(2,4-dichloro-5-fluorobenzoyl)acrylate.

3. Ethyl 3-dimethylamino-2-(2,4-dichloro-5-fluorobenzoyl)acrylate.

4. 3-Dimethylamino-2-(2,3,4,5-tetrafluorobenzoyl)-acrylonitrile.

5. 3-Dimethylamino-2-(2,4-dichloro-5-fluorobenzoyl)-acrylonitrile.

6. Process for the preparation of 3-amino-2-benzoyl-acrylic acid derivatives of the formula I

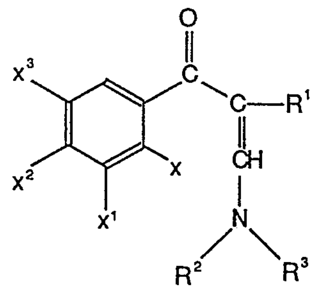

I

in which

$R^1$ represents the nitrile group, an ester group $-COOR^4$ or carboxamide group

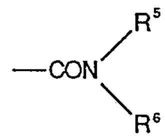

$R^4$ denoting $C_1$–$C_6$-alkyl, and

$R^5$ and $R^6$ representing hydrogen or $C_1$–$C_3$-alkyl or phenyl,

$R^2$ and $R^3$, which can be identical or different, represent a $C_1$–$C_6$-alkyl radical and, furthermore, can form, together with the nitrogen atom to which they are bonded, a 5- or 6-membered heterocyclic ring which can additionally contain as ring member the atoms or groups $-O-$, $-S-$, $-SO-$ or $-SO_2-$,

X represents halogen,

$X^1$ denotes hydrogen, methyl, nitro or halogen,

$X^2$ denotes halogen or methyl, and

$X^3$ denotes hydrogen or halogen,

excluding ethyl 2-(2′,4′-dichlorobenzoyl)-3-dimethylamino-propenoate,

characterised in that benzoyl halides of the formula II

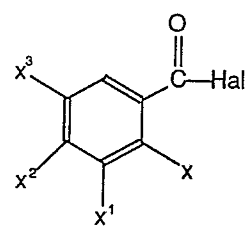

II

in which

Hal denotes halogen, and

X, $X^1$, $X^2$ and $X^3$ have the above mentioned meaning, are reacted with 3-aminoacrylic acid derivatives of the formula III

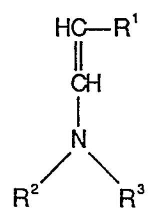

III

in which

$R^1$, $R^2$ and $R^3$ have the above mentioned meaning, where appropriate in the presence of an acid acceptor.

7. Process according to Claim 6, characterised in that 2,4-dichloro-5-fluorobenzoyl chloride is reacted with methyl 3-dimethylaminoacrylate.

8. Process according to Claim 6, characterised in that 2,4-dichloro-5-fluorobenzoyl chloride is reacted with ethyl 3-dimethylaminoacrylate.

9. Process according to Claim 6, characterised in that 2,4-dichloro-5-fluorobenzoyl chloride is reacted with 3-dimethylaminoacrylonitrile.

10. Process according to Claim 6, characterised in that an organic base or sodium hydride is used as the acid acceptor.

11. Use of the 3-amino-2-benzoylacrylic acid derivatives of the formula I in Claim 1, including the compound excluded in claim 1, as intermediates for the synthesis of antibacterial medicaments from the group of quinolone-carboxylic acids.

**Revendications**

1. Dérivés d'acide 3-amino-2-benzoyl-acrylique de formula I

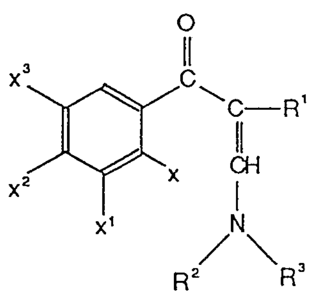

I

dans laquelle
$R^1$ représente le groupe nitrile, un groupe ester $-COOR^4$ ou un groupe carboxamide

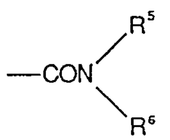

où
$R^4$ est un groupe alkyle en $C_1$ à $C_6$ et
$R^5$ et $R^6$ représentent l'hydrogène, un groupe alkyle en $C_1$ ou $C_3$ ou le groupe phényle,
$R^2$ et $R^3$ peuvent être identiques ou différents et représentent un reste alkyle en $C_1$ à $C_6$ et peuvent former en outre conjointement avec l'atome d'azote auquel ils sont liés, un noyau hétérocyclique pentagonal ou hexagonal qui peut comporter en outre comme chaînon cyclique les atomes ou les groupes -O-, -S-, -SO- ou $-SO_2-$,
X est un halogène,
$X^1$ est l'hydrogène, le groupe méthyle, le groupe nitro ou un halogène
$X^2$ est un halogène ou le groupe méthyle et
$X^3$ est l'hydrogène ou un halogène,
excepté le 2-(2',4'-dichlorobenzoyl)-3-diméthylaminopropénoate d'éthyle.

2. L'ester méthylique de l'acide 3-diméthylamino-2-(2,4-dichloro-5-fluorobenzoyl)acrylique.

3. L'ester éthylique de l'acide 3-diméthylamino-2-(2,4-dichloro-5-fluorobenzoyl)acrylique.

4. Le 3-diméthylamino-2-(2,3,4,5-tétrafluorobenzoyl)acrylonitrile.

5. Le 3-diméthylamino-2-(2,4-dichloro-5-fluorobenzoyl)acrylonitrile.

6. Procédé de production de dérivés d'acide 3-amino-2-benzoylacrylique de formule I

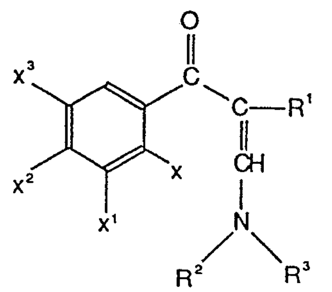

I

dans laquelle
$R^1$ représente le groupe nitrile, un groupe ester $-COOR^4$ ou un groupe carboxamide

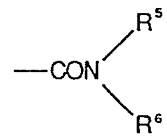

où
$R^4$ est un groupe alkyle en $C_1$ à $C_6$ et
$R^5$ et $R^6$ représentent l'hydrogène, un groupe alkyle en $C_1$ ou $C_3$ ou le groupe phényle,
$R^2$ et $R^3$ peuvent être identiques ou différents et représentent un reste alkyle en $C_1$ à $C_6$ et peuvent former en outre conjointement avec l'atome d'azote auquel ils sont liés, un noyau hétérocyclique pentagonal ou hexagonal qui peut comporter en outre comme chaînon cyclique les atomes ou les gorupes -O-, -S-, -SO- ou $-SO_2-$,
X est un halogène,
$X^1$ est l'hydrogène, le groupe méthyle, le groupe nitro ou un halogène
$X^2$ est un halogène ou le groupe méthyle et
$X^3$ est l'hydrogène ou un halogène,
excepté le 2-(2',4'-dichlorobenzoyl)-3-diméthylaminopropénoate d'éthyle,
caractérisé en ce qu'on fait réagir des halogénures de benzoyle de formule II

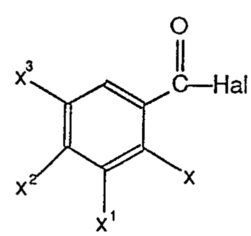

II

dans laquelle Hal désigne un halogène et X, $X^1$, $X^2$ et $X^3$ ont la définition indiquée ci-dessus, avec

des dérivés d'acide 3-aminoacrylique de formule III

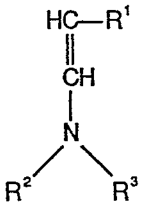

III

dans laquelle $R^1$, $R^2$ et $R^3$ ont la définition indiquée cidessus, le cas échéant en présence d'un accepteur d'acide.

7. Procédé suivant la revendication 6, caractérisé en ce qu'on fait réagir le chlorure de 2,4-dichloro-5-fluorobenzoyle avec l'ester méthylique d'acide 3-diméthylamino-acrylique.

8. Procédé suivant la revendication 6, caractérisé en ce qu'on fait réagir le chlorure de 2,4-dichloro-5-fluorobenzoyle avec l'ester éthylique d'acide 3-diméthylamino-acrylique.

9. Procédé suivant la revendication 6, caractérisé en ce qu'on fait réagir le chlorure de 2,4-dichloro-5-fluorobenzoyle avec le 3-diméthyl amino-acrylonitrile.

10. Procédé suivant la revendication 6, caractérisé en ce qu'on utilise comme accepteur d'acide une base organique ou l'hydrure de sodium.

11. Utilisation des dérivés d'acide 3-amino-2-benzoyl-acrylique de formule I suivant la revendication 1, y compris le composé excepté dans la revendication 1, comme produits itnermédiaires pour la synthèse de médicaments antibactériens du groupe des acides quinolone-carboxyliques.